(19) **Europäisches Patentamt**
**European Patent Office**
**Office européen des brevets**

(11) **EP 4 014 951 A1**

(12) **EUROPEAN PATENT APPLICATION**
published in accordance with Art. 153(4) EPC

(43) Date of publication:
**22.06.2022 Bulletin 2022/25**

(21) Application number: **20860323.3**

(22) Date of filing: **13.04.2020**

(51) International Patent Classification (IPC):
*A61K 8/02* (2006.01)     *A61K 8/98* (2006.01)
*A61K 8/14* (2006.01)     *A61K 8/60* (2006.01)
*A61K 8/67* (2006.01)     *A61Q 19/00* (2006.01)

(86) International application number:
**PCT/KR2020/004966**

(87) International publication number:
**WO 2021/045343 (11.03.2021 Gazette 2021/10)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **06.09.2019 KR 20190110546**

(71) Applicant: **BN Co., Ltd.**
**Gyeonggi-do 14059 (KR)**

(72) Inventors:
• **KIM, Bong Woo**
**Cheongju-si Chungcheongbuk-do 28165 (KR)**

• **SHIM, Hong Bo**
**Cheongju-si Chungcheongbuk-do 28774 (KR)**
• **KIM, Jae Hwan**
**Sejong 30064 (KR)**
• **RHIM, Ji Heon**
**Cheongju-si Chungcheongbuk-do 28424 (KR)**
• **RYOO, Jae Duck**
**Cheongju-si Chungcheongbuk-do 28472 (KR)**

(74) Representative: **Prüfer & Partner mbB**
**Patentanwälte · Rechtsanwälte**
**Sohnckestraße 12**
**81479 München (DE)**

(54) **STRUCTURE FOR PROMOTING TRANSDERMAL ABSORPTION, PREPARATION METHOD THEREFOR, AND COSMETIC COMPOSITION COMPRISING SAME**

(57)     The present invention relates to a structure for accelerating transdermal absorption, a manufacturing method thereof, and a cosmetic composition comprising the same, and the structure for accelerating transdermal absorption comprises an acicular body; a hydrophilic group binded to the acicular body; and a carrier binded to the hydrophilic group.

【Figure 1】

## Description

[Technical Field]

The present invention relates to a structure for accelerating transdermal absorption, which can accelerate transdermal absorption of an active ingredient, a method of manufacturing the structure for accelerating transdermal absorption, and a cosmetic composition comprising the structure for accelerating transdermal absorption.

[Background Art]

In general, scars rarely remain even when skin damage occurs as a child, whereas scars easily remain when skin damage occurs with age, which is predicted to be because the proliferation rate of skin keratinocyte stem cells and transit-amplifying cells required for skin regeneration is remarkably lowered by skin aging. That is, as the skin aging progresses, the proliferation rate of various cells decreases, thereby resulting in a decrease in skin regeneration ability, the wrinkle formation, the blemish formation, a reduction in skin elasticity, and the like.

Accordingly, techniques for various skin care methods or cosmetic compositions for preventing skin aging or maintaining or improving the current level of skin condition have been proposed.

Specifically, Korean Patent Laid-open No. 2010-0104703 relates to a functional cosmetic composition for renewing skin and a method for using the same, and discloses that it is possible to facilitate the transdermal absorption, lower the cytotoxicity, and have the skin regeneration effect by using 5-aminolevulinic acid ester or a salt thereof as an active ingredient. In addition, Korean Patent Laid-open No. 2011-0119062 relates to an enriched media of human adipose tissue-derived stem cells and uses thereof, and discloses that it is possible to have skin regeneration or wrinkle improvement effect by using an enriched media of human adipose tissue-derived stem cells as an active ingredient. In addition, Korean Patent Laid-open No. 2018-0134468 relates to cosmetic compositions comprising spicule, marine collagen and deep ocean water, and their preparation, and discloses that it is possible to obtain wrinkle improvement, pigmentation alleviation, acne improvement, or new skin regeneration effect by using deep ocean water and marine collagen extract rich in various minerals.

However, the above techniques have a problem that the effect of preventing skin aging or maintaining and improving the skin condition is not large because the transdermal absorption rate of the active ingredient is low and there is a limit to the deep penetration of the active ingredient into the skin.

[Disclosure]

[Technical Problem]

It is an object of the present invention to provide a structure for accelerating transdermal absorption, which can increase the transdermal absorption rate of an active ingredient and deeply penetrate an active ingredient into the skin.

It is another object of the present invention to provide a method of manufacturing the structure for accelerating transdermal absorption.

It is still another object of the present invention to provide a cosmetic composition comprising the structure for accelerating transdermal absorption.

[Technical Solution]

To solve the above problems, the present invention provides a structure for accelerating transdermal absorption, comprising: an acicular body; a hydrophilic group binded to the acicular body; and a carrier binded to the hydrophilic group.

In addition, the present invention provides a method of manufacturing a structure for accelerating transdermal absorption, comprising the steps of: washing an acicular body; reacting the washed acicular body with a basic compound to bind a hydrophilic group to the acicular body; and mixing a carrier and the acicular body to which the hydrophilic group is binded, thereby binding the carrier to the hydrophilic group.

In addition, the present invention provides a cosmetic composition comprising: the structure for accelerating transdermal absorption; and an active ingredient contained in the structure for accelerating transdermal absorption, wherein the active ingredient is one or more selected from the group consisting of nicotinamide adenine dinucleotide ($NAD^+$), nicotinamide mononucleotide (NMN), nicotinamide (NAM), nicotinamide riboside (NR), retinol, retinyl acetate, retinyl palmitate, acetyl hexapeptide-3 (Ac-EEMQRR-$NH_2$), pentapeptide-3 (GPRPA-$NH_2$), pentapeptide-18 (Y(dA)GFL-$NH_2$), palmitoyl tripeptide-1 (pal-GHK-$NH_2$), palmitoyl pentapeptide-4 (pal-KTTKS-$NH_2$), and hexapeptide-11 (FVAPFP-$NH_2$).

[Advantageous Effects]

The structure for accelerating transdermal absorption of the present invention may be stably binded to a large amount of carriers as its surface comprises an acicular body modified with hydrophilicity. Therefore, when the structure for accelerating transdermal absorption of the present invention obtained by binding a carrier containing an active ingredient to an acicular body is used in a cosmetic composition, it is possible to provide a cosmetic composition having an excellent functionality (for example, anti-aging function) since the transdermal absorption rate of the active ingredient can be increased and the active ingredient can be deeply penetrated into the skin.

**[Brief Description of Drawings]**

FIG. 1 is a schematic view showing the structure for accelerating transdermal absorption according to an example of the present invention.
FIG. 2 is a reference view for explaining the results according to Experimental Example 1 of the present invention.
FIG. 3 is a reference view for explaining the results according to Experimental Example 2 of the present invention.
FIGS. 4 and 5 are reference views for explaining the results according to Experimental Example 3 of the present invention.
FIG. 6 is a reference view for explaining the results according to Experimental Example 4 of the present invention.
FIG. 7 is a reference view for explaining the results according to Experimental Example 5 of the present invention.

**[Best Mode]**

The terms and words as used in the description and claims of the present invention should not be construed as limited to conventional or dictionary meanings, but should be construed as the meaning and concept consistent with the technical idea of the present invention based on the principle that the inventor can appropriately define the concept of the terms to describe its own invention in the best way.

The present invention relates to a structure capable of accelerating skin absorption of an active ingredient in absorbing the active ingredient, which functions to prevent skin aging or to maintain and improve skin condition, to the skin. Specifically, the present invention relates to a structure for accelerating transdermal absorption having both a function of accelerating physical transdermal absorption and a function of accelerating formulation transdermal absorption, which will be described with reference to the drawings as follows:

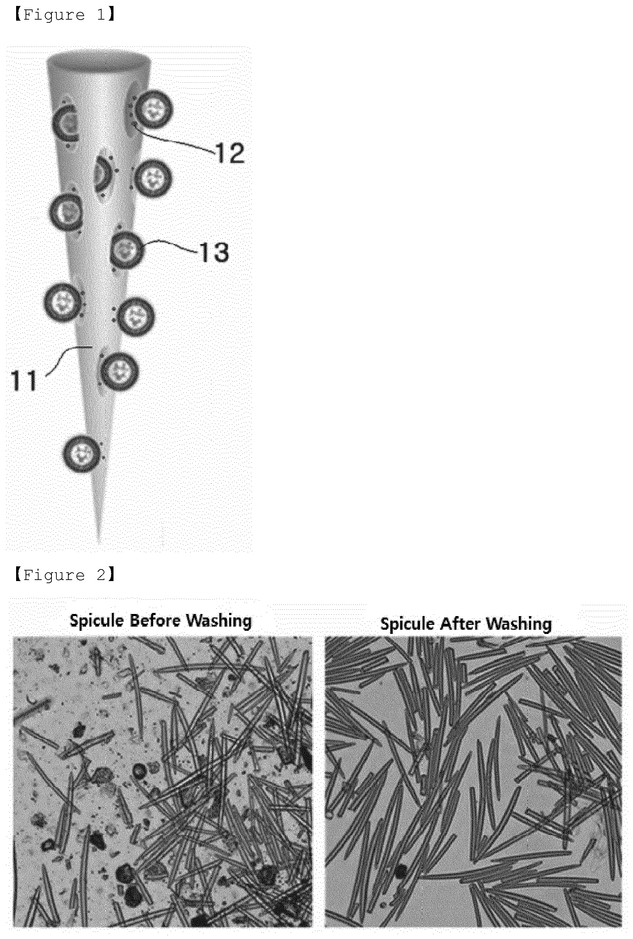

Referring to FIG. 1, the structure for accelerating transdermal absorption according to an example of the present invention (hereinafter referred to as "structure") comprises an acicular body 11, a hydrophilic group 12, and a carrier 13.

The acicular body 11 included in the structure according to an example of the present invention may have at least a portion having an acicular shape (for example, a needle shape, a cone shape, and a pyramid shape). If the acicular body 11 is dispersed in the skin, it may perform a function of forming a hole in the skin, thereby accelerating physical transdermal absorption of the active ingredient. In addition, since the acicular body 11 may also perform a function of penetrating into the skin to push up and exfoliate the dead skin cells accumulated in the skin, it may also promote the process of skin regeneration.

The material of the acicular body 11 is not particularly limited as long as it is harmless to the human body, but may be a spicule extracted from porifera. As the material of the acicular body 11 is a spicule, it is possible to increase the productivity of the cosmetic composition while minimizing irritation to the skin. In addition, as the spicule is porous, the impregnation of the active ingredient can be efficiently made to increase the functionality of the cosmetic composition.

The hydrophilic group 12 included in the structure according to an example of the present invention is binded to the acicular body 11 (specifically dispersed on and binded to the surface of the acicular body 11), and may perform a function of stably binding the carrier 13 to the acicular body 11.

The hydrophilic group 12 is not particularly limited, but may be a hydroxyl group (-OH). As the hydrophilic group 12 is a hydroxyl group, it is possible to minimize irritation to the skin while increasing the binding force with the carrier 13.

The carrier 13 included in the structure according to an example of the present invention is binded to the hydrophilic group 12, and may contain an active ingredient to perform a function of transporting and delivering the active ingredient into the skin. The carrier 13 may consist of an organic material or an inorganic material. Specifically, the carrier 13 may be an organic material such as a polymer hydrogel, a polymer micelle, a nanoemulsion (size: 100 to 500 nm), a liposome, an oleosome, a niosome, an ethosome, and the like, or an inorganic material such as a talc, a bentonite, a silica, and the like.

The polymer hydrogel may be a material in which a phase transition occurs from a sol to a gel or a gel to a sol. Specifically, the polymer hydrogel may be a material comprising one or more gelling polymers selected from the group consisting of galactomannan, glucomannan, guar gum, locust bean gum, *Ceratonia siliqua* gum, pluronic, agar, algin, carrageenan, xanthan gum, and gellan gum.

The polymer micelle may be a block copolymer material in which a hydrophilic polymer such as polyalkylene glycol and the like, and a hydrophobic polymer such as polylactide, polyglycolide, polycaprolactone and the like are bonded.

The liposome has a lipid bilayer structure that is structurally similar to the cell membrane or the intercellular lipid of the skin epidermal layer, and may be a material that is hydrophobic inside and hydrophilic outside.

More specifically, the carrier 13 may be a liposome having a hollow therein. The hollow (formed) present inside the liposome may contain an active ingredient to perform a function of delivering the active ingredient into the skin. The liposome may perform a function of delivering the active ingredient to the dermis layer of the skin, thereby accelerating the formulation transdermal absorption of the active ingredient.

The liposome may comprise one or more lipids selected from the group consisting of soybean lecithin (L-$\alpha$-lecithin), lysolecithin, sphingomyelin, phosphatidylcholine, phosphatidylserine, phosphatidylglycerol, phosphatidylinositol, phosphatidic acid, phosphatidylethanolamine, dipalmitoyl phosphatidylcholine (DPPC), dipalmitoyl phosphatidylglycerol (DMPG), and dipalmitoyl phosphatidic acid (DPPA).

The carrier 13 may be binded to the acicular body 11 with a higher binding force by interconnection with the hydrophilic group 12 of the acicular body 11. The interconnection is not particularly limited, but may be a hydrogen bond or a covalent bond. The carrier 13 may be hydrophilic, and thus increase the transdermal absorption rate of the active ingredient.

Specifically, if the acicular body 11 is porous, the carrier 13 may be binded to the pores of the acicular body 11 through inclusion, and in this case, since the binding force is not high, the carrier 13 may be easily separated from the acicular body 11 during the manufacturing process of the cosmetic composition or the use process of the cosmetic composition. At this time, if the carrier 13 is hydrophilic as in the hydrophilic group 12 binded to the acicular body 11, the carrier 13 may be stably binded to the acicular body 11 by the interaction between the hydrophilic group 12 binded to the acicular body 11 and the carrier 13, and thus the carrier 13 may be prevented from being easily separated from the acicular body 11 during the manufacturing process of the cosmetic composition or the use process of the cosmetic composition. Wherein, if an active ingredient is contained in the carrier 13, the loss of the carrier 13 in the process of transdermal absorption is minimized by stable bond between the acicular body 11 and the carrier 13, thereby increasing the transdermal absorption rate of the active ingredient.

Along with the case where all of the carriers 13 are binded to the hydrophilic group 12 of the acicular body 11, the case where some of the carriers 13 are binded to the hydrophilic group 12 of the acicular body 11 and others of the carriers 13 are binded to the surface of the acicular body 11 to which the hydrophilic group 12 is not binded may be also included in the scope of the present invention.

The structure according to an example of the present invention may have a high porosity so that the inclusion of the carrier 13 is efficiently performed. That is, the structure according to an example of the present invention may have a porosity of 10 to 95%, specifically 20 to 95%, more specifically 30 to 90%, still more specifically 50 to 55%. As the porosity is within the above range, it is possible to maintain the physical strength of the structure while increasing the inclusion rate of the carrier 13. Wherein, the porosity may be a value calculated by the following equation:

$$[\text{Equation}]$$

$$\text{Porosity} = \{(W_1-W_2)/W_1\} \times 100$$

wherein, $W_1$ is the initial weight of the acicular body that is not subjected to the process of pore formation in the manufacturing process of the structure (for example, the weight of the spicules before pore formation); and
$W_2$ is the weight of the acicular body that has been subjected to the process of pore formation in the manufacturing process of the structure (for example, the weight of the spicules after pore formation).

The present invention provides a method for manufacturing the structure as described above and its specific description is as follows:

First, the method of manufacturing the structure according to an example of the present invention comprises the step of washing an acicular body. The washing is for removing impurities present in an acicular body and may be performed using purified water and an acidic solution. Specifically, the washing of an acicular body may be subjected to the process of washing an acicular body with purified water one or more times, and then treating it with an acidic solution such as hydrogen peroxide solution. Wherein, upon treating the acicular body with the acidic solution, ultrasonic waves may be applied. As the ultrasonic waves are applied, the washing efficiency of the acicular body may be increased.

Next, the method of manufacturing the structure according to an example of the present invention comprises the step of reacting the washed acicular body with a basic compound to bind a hydrophilic group to the acicular body. The step is the step of modifying the surface of the acicular body, wherein the basic compound used is not particularly limited, but may be one or more selected from the group consisting of sodium hydroxide, potassium hydroxide, and lithium hydroxide. As the basic compound is the listed compound, the binding efficiency (dispersion efficiency) of the hydrophilic group may be increased. Wherein, the reaction of the acicular body and the basic compound may be performed at 25 to 50°C for 30 minutes to 2 hours so that the hydrophilic group is well binded to the acicular body.

Next, the method for manufacturing the structure according to an example of the present invention comprises the step of mixing a carrier and the acicular body to which the hydrophilic group is binded, thereby binding the carrier to the hydrophilic group. Wherein, upon mixing the acicular body and the carrier so that the hydrophilic group and the carrier may be well binded, an ultrahigh pressure treatment in which a pressure of 50 to 300 MPa is applied for 1 to 10 minutes may be performed.

The method for manufacturing the structure according to an example of the present invention may further comprise the step of forming pores capable of enhancing the porosity of the acicular body. Specifically, prior to reacting the washed acicular body with the basic compound to bind the hydrophilic group to the acicular body, one or more pores are formed in the acicular body by reacting the washed acicular body with an acidic solution (hydrogen peroxide solution). As the process of forming pores is performed, the porosity of the acicular body is enhanced (the number of pores in the acicular body is increased), thereby increasing the inclusion rate of the carrier. Wherein, the reaction between the acicular body and the acidic solution (hydrogen peroxide solution) may be performed at room temperature for 6 to 20 hours. In addition, when the acicular body and the acidic solution (hydrogen peroxide solution) are reacted so that the reaction between the acicular body and the acidic solution (hydrogen peroxide solution) can be activated to efficiently form pores, ultrasonic waves having a frequency of 10 to 70 kHz and an output of 100 to 500 W may be applied.

The process of reacting the acicular body with the acidic solution (hydrogen peroxide solution) to form pores may be repeated one or more times, and applying ultrasonic waves for each process may be selectively performed.

As the structure is manufactured by the manufacturing method as described above, the present invention may provide a structure in which a large amount of carriers are stably binded to the acicular body. Therefore, if the structure of the present invention is used for transdermal absorption of the active ingredient, it is possible to obtain the effect capable of increasing the transdermal absorption rate while accelerating transdermal absorption.

Meanwhile, the present invention provides a cosmetic composition comprising the structure as described above and an active ingredient.

The structure included in the cosmetic composition according to an example of the present invention contains an active ingredient, and its specific description is as described above and thus will be omitted. Wherein, the active ingredient may be contained on the surface of the acicular body included in the structure and/or inside the carrier, and specifically, may be contained inside the carrier.

The content of the structure is not particularly limited, but may be 0.1 to 1 part by weight based on 100 parts by weight of the cosmetic composition.

The active ingredient contained in the structure is not particularly limited, but may be one or more selected from the group consisting of nicotinamide adenine dinucleotide ($NAD^+$), nicotinamide mononucleotide (NMN), nicotinamide (NAM), nicotinamide riboside (NR), retinol, retinyl acetate, retinyl palmitate, acetyl hexapeptide-3 (Ac-EEMQRR-$NH_2$), pentapeptide-3 (GPRPA-$NH_2$), pentapeptide-18 (Y(dA)GFL-$NH_2$), palmitoyl tripeptide-1 (pal-GHK-$NH_2$), palmitoyl pentapeptide-4 (pal-KTTKS-$NH_2$), and hexapeptide-11 (FVAPFP-$NH_2$), which have an excellent anti-aging function.

The content of the active ingredient is not particularly limited, but may be 0.05 to 1 part by weight based on 100 parts by weight of the cosmetic composition.

The cosmetic composition according to an example of the present invention may further comprise purified water, an antioxidant, a stabilizer, a pigment, a perfume, or the like, which is commonly used in field of cosmetic compositions.

In addition, the cosmetic composition according to an example of the present invention may be prepared into a conventional formulation. Specifically, it may be formulated into a solution, a suspension, an emulsion, a paste, a gel, a cream, a lotion, a powder, an emulsion foundation, a wax foundation, a spray, or the like.

Hereinafter, the present invention will be described in more detail by examples. However, the following examples are only for illustrating the present invention, which will be apparent to those skilled in the art that various changes and modifications may be made within the category and spirit of the present invention, and the scope of the present invention is not limited thereto.

**[Example** 1]

1) Washing of Spicule

A spicule (J2KBIO Co., Ltd.) extracted (obtained) from *Spongilla lacustris* L. was used as an acicular body, and was washed through the following processes: 20 g of spicule was put in 200 ml of purified water to remove salt, and filtered through a 400 mesh polyethylene (PE) filter, and then the spicule left on the filter was washed with purified water two times. Subsequently, the washed spicule was put in a dryer (Hanbaek Co., Ltd.) and dried at 50 °C.

To remove impurities remaining in the dried spicule, the dried spicule was put in 200 ml of 35% hydrogen peroxide solution ($H_2O_2$) (Samchun Chemical Co. Ltd.) and subjected to ultrasonication under conditions of 300 W and 40 kHz for 1 hour using an ultrasonic cleaner (Sungdong Ultrasonic Co., Ltd.). Then, after filtering it through a 400 mesh polyethylene (PE) filter, the process of washing the spicule left on the filter with 500 ml of purified water was repeated three times. Subsequently, the process of putting the washed spicule into a dryer and drying it at 50 °C was performed to complete the washing of the spicule.

2) Binding of Hydroxyl Group to Spicule

200 ml of 1N NaOH (Sigma-Aldrich Co. Ltd.) was added to 20 g of the pore-formed spicule, and reacted at 40 °C for 1 hour. Then, after filtering it through a 400 mesh polyethylene (PE) filter, the process of washing the spicule left on the filter with 500 ml of purified water was repeated three times. Subsequently, the process of putting the washed spicule into a dryer and drying it at 50 °C was performed to obtain the spicule to which a hydrophilic hydroxyl group was binded.

3) Binding of Liposome to Spicule

0.5 g (5%) of the hydroxyl group-binded spicule and 1 g (10%) of the fluorescent protein liposome as a carrier were added to 8.5 ml of purified water, and the spicule and the fluorescent protein liposome were mixed by stirring at room temperature for 1 hour to prepare a mixed solution containing a spicule-liposome binding structure. At this time, to a solution that was obtained by dispersing 1 g of L-α-lecithin in 6 g of 1,3-butylene glycol (Sigma Co., Ltd.), adding 6 g of purified water, heating it in a hot water bath at 80 °C to dissolve it, and cooling it to 50 °C, 1 g of purified water, in which Alexafluor™ 488-binding protein (Thermo Fisher Scientific Co. Ltd.) was dissolved at a concentration of 5 mg/ml, was added in small portions, thereby preparing a mixture, in which these solutions were uniformly mixed with each other, and then 6 g of purified water was added to the mixture in small portions again, stirred at room temperature, and refrigerated at 4 °C to use as the fluorescent protein liposome.

Next, after filtering the prepared mixed solution through a 400 mesh polyethylene (PE) filter, the process of washing the spicule-liposome binding structure left on the filter with 500 ml of purified water was repeated three times. Subsequently, the process of putting the washed spicule-liposome binding structure into a dryer and drying it at 50 °C was performed to obtain a structure, in which the fluorescent protein liposome was binded to the pores of the spicule and the hydroxyl group of the spicule.

**[Example 2]**

A structure was obtained by performing the same process as in Example 1, except that the process of putting the washed spicule into 200 ml of 35% hydrogen peroxide solution ($H_2O_2$) (Samchun Chemical Co. Ltd.) and reacting them for 16 hours to form the pores in the spicule was further carried out (the porosity of the spicule is enhanced).

**[Example 3]**

A structure was obtained by performing the same process as in Example 2, except that prior to binding the hydroxyl group to the spicule, the process of putting the pore-formed spicule into 200 ml of 35% hydrogen peroxide solution ($H_2O_2$) (Samchun Chemical Co. Ltd.) and being subjected to ultrasonication under conditions of 300 W and 40 kHz for 1 hour using an ultrasonic cleaner was further carried out (addition of one ultrasonication compared to Example 2).

**[Example 4]**

A structure was obtained by performing the same process as in Example 3, except that the process of putting the spicule, which was further subjected to the ultrasonication process, into 200 ml of 35% hydrogen peroxide solution ($H_2O_2$) (Samchun Chemical Co. Ltd.) and being subjected to ultrasonication under conditions of 300 W and 40 kHz for 1 hour using an ultrasonic cleaner was carried out again (addition of two ultrasonications compared to Example 2).

**[Example 5]**

A structure was obtained by performing the same process as in Example 2, except that the process of putting 10 g of a mixed solution containing a spicule-liposome binding structure into a plastic pack and vacuum-reducing it, and then pressurizing for 5 minutes at a pressure of 150 MPa using a high-pressure processing machine (Ilshin Autoclave Co., Ltd., Suflux®) was further carried out.

**[Example 6]**

A structure was obtained by performing the same process as in Example 3, except that the process of putting 10 g of a mixed solution containing a spicule-liposome binding structure into a plastic pack and vacuum-reducing it, and then pressurizing for 5 minutes at a pressure of 150 MPa using a high-pressure processing machine (Ilshin Autoclave Co., Ltd., Suflux®) was further carried out.

**[Example 7]**

A structure was obtained by performing the same process as in Example 4, except that the process of putting 10 g of a mixed solution containing a spicule-liposome binding structure into a plastic pack and vacuum-reducing it, and then pressurizing for 5 minutes at a pressure of 150 MPa using a high-pressure processing machine (Ilshin Autoclave Co., Ltd., Suflux®) was further carried out.

**[Comparative Example 1]**

A structure was obtained by performing the same process as in Example 1, except that the process of binding a hydroxyl group was not carried out.

**[Comparative Example 2]**

A structure was obtained by performing the same process as in Example 2, except that the process of binding a hydroxyl group was not carried out.

**[Comparative Example 3]**

A structure was obtained by performing the same process as in Example 3, except that the process of binding a hydroxyl group was not carried out.

**[Comparative Example 4]**

A structure was obtained by performing the same process as in Example 4, except that the process of binding a hydroxyl group was not carried out.

**[Comparative Example 5]**

The fluorescent protein liposome, which is the carrier used in Example 1, was applied (the fluorescent protein liposome, to which the spicule was not binded, was applied alone).
The conditions of the Examples and Comparative Examples above are summarized as in Table 1 below.

[Table 1]

| Condition | Washing of spicule | Pore formation of spicule | Binding of hydroxyl group | Pressurization upon binding of liposome | Porosity of spicule (%) |
|---|---|---|---|---|---|
| Comparative Example 1 | $H_2O_2$ 1 hour ultrasonication | - | - | - | 25.0% |
| Comparative Example 2 | $H_2O_2$ 1 hour ultrasonication | $H_2O_2$ (16 hours) | - | - | 50.0% |
| Comparative Example 3 | $H_2O_2$ 1 hour ultrasonication | $H_2O_2$ (16 hours)+$H_2O_2$ (1 hour ultrasonication) | - | - | 52.0% |

(continued)

| Condition | Washing of spicule | Pore formation of spicule | Binding of hydroxyl group | Pressurization upon binding of liposome | Porosity of spicule (%) |
|---|---|---|---|---|---|
| Comparative Example 4 | $H_2O_2$ 1 hour ultrasonication | $H_2O_2$ (16 hours)+$H_2O_2$ (1 hour ultrasonication) +$H_2O_2$ (1 hour ultrasonication) | - | - | 54.8% |
| Comparative Example 5 | - | - | - | - | - |
| Example 1 | $H_2O_2$ 1 hour ultrasonication | - | NaOH 1 hour | - | 25.0% |
| Example 2 | $H_2O_2$ 1 hour ultrasonication | $H_2O_2$ (16 hours) | NaOH 1 hour | - | 50.0% |
| Example 3 | $H_2O_2$ 1 hour ultrasonication | $H_2O_2$ (16 hours)+$H_2O_2$ (1 hour ultrasonication) | NaOH 1 hour | - | 52.0% |
| Example 4 | $H_2O_2$ 1 hour ultrasonication | $H_2O_2$ (16 hours)+$H_2O_2$ (1 hour ultrasonication) +$H_2O_2$ (1 hour ultrasonication) | NaOH 1 hour | - | 54.8% |
| Example 5 | $H_2O_2$ 1 hour ultrasonication | $H_2O_2$ (16 hours) | NaOH 1 hour | 150 MPa, 5 minutes | 50.0% |
| Example 6 | $H_2O_2$ 1 hour ultrasonication | $H_2O_2$ (16 hours)+$H_2O_2$ (1 hour ultrasonication) | NaOH 1 hour | 150 MPa, 5 minutes | 52.0% |
| Example 7 | $H_2O_2$ 1 hour ultrasonication | $H_2O_2$ (16 hours)+$H_2O_2$ (1 hour ultrasonication) +$H_2O_2$ (1 hour ultrasonication) | NaOH 1 hour | 150 MPa, 5 minutes | 54.8% |

**[Experimental Example 1]**

To confirm whether the washing of the spicule was performed well, the spicule before washing and the spicule after washing in Example 1 were checked with an optical microscope (Nikon Corporation, ECLIPSE TS2), and the results are shown in FIG. 2.

Referring to FIG. 2, it may be confirmed that the washing of the spicule was well performed.

**[Experimental Example 2]**

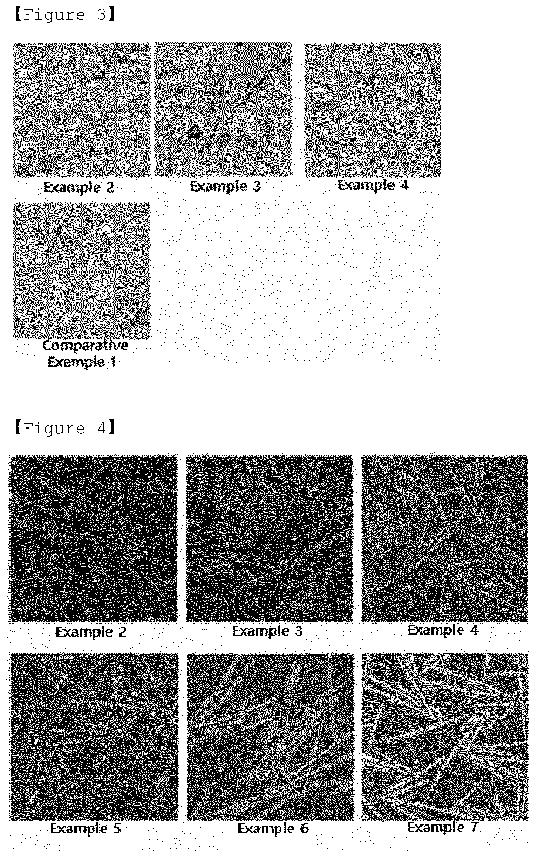

The porosity evaluation for the spicules of Examples 2 to 4, in which washing, pore formation, and/or ultrasonication were performed until the hydroxyl group was binded to the spicule, and the spicule of Comparative Example 1, in which only the washing process was performed, was carried out in the following manner, and the results are shown in FIG. 3 and Table 2 below:

- Porosity Evaluation of Spicule: The number of spicules was measured by performing the process of putting 5 mg of the spicule into 500 mg of purified water, mixing them to prepare a mixed solution, and then taking 10 mg from the prepared mixed solution to put it into a hemocytometer, and observing it with an optical microscope (Nikon Corporation, ECLIPSE TS2, at 40 magnification).

Referring to FIG. 3, it may be confirmed that the number of spicules in Examples 2 to 4, in which washing, pore formation, and ultrasonication were performed, is higher compared to Comparative Example 1, in which only the washing process was performed. This is because, in the case of Examples 2 to 4, as the porosity of the spicule increases and thus the weight of the spicule decreases, the number of spicules confirmed per unit weight is high.

[Table 2]

| Classification | Weight of spicules after washing (g) | Weight per 1 spicule (mg) |
|---|---|---|
| Comparative Example 1 | 9 | 0.000045 |
| Example 2 | 8.5 | 0.0000425 |
| Example 3 | 6.4 | 0.000032 |
| Example 4 | 6.2 | 0.000031 |

Referring to Table 2 above, it can be confirmed that the weight of the spicules in Examples 2 to 4, in which washing, pore formation, and ultrasonication were performed, was significantly reduced compared to Comparative Example 1, in which only the washing process was performed. This can be seen as a result of supporting that as the pore formation and ultrasonication for the spicule are performed as described in the present invention, the porosity of the spicule becomes very high.

**[Experimental Example 3]**

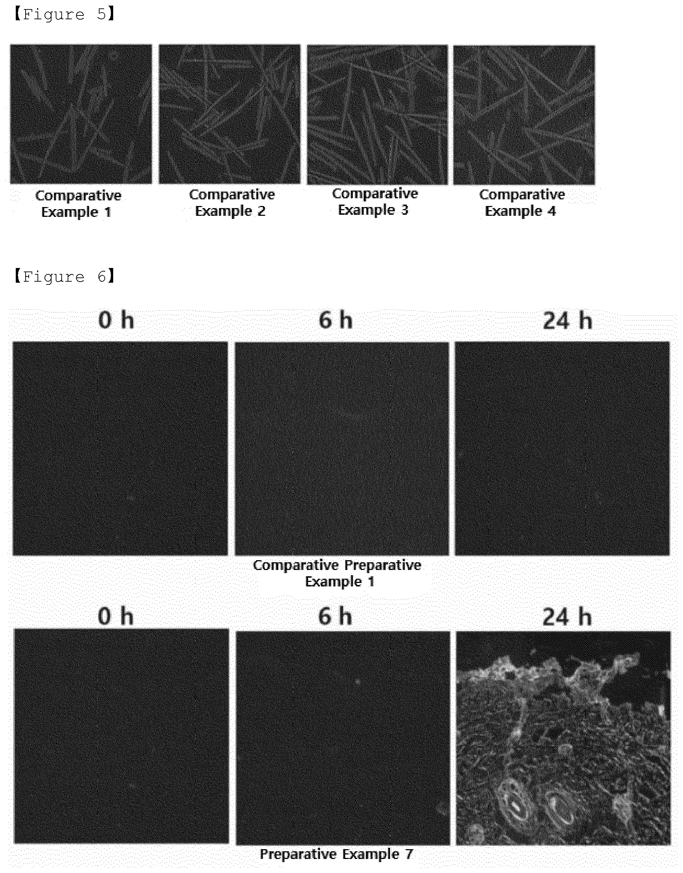

To confirm whether the inclusion of the fluorescent protein liposomes was well performed in the structures obtained in Examples 2 to 7 and Comparative Examples 1 to 4, 5 mg of the structure was put into 500 mg of purified water and mixed to prepare a mixed solution, and then 100 mg was taken from the prepared mixed solution, placed on a slide glass, and observed with a fluorescence microscope (NEXCOPE Co., Ltd., NIB410F) GFP filter, and the results are shown in FIGS. 4 and 5.
Referring to FIGS. 4 and 5, it can be confirmed that Examples 2 to 7 have better binding of the fluorescent protein liposome compared to Comparative Examples 1 to 4.

**[Preparative Examples 1 to 7]**

Cosmetic compositions were prepared using the respective structures obtained in Examples 1 to 7. Specifically, based on 100 parts by weight of the cosmetic composition, 2.0 parts by weight of glycerin (moisturizer), 3.0 parts by weight of panthenol (moisturizer), 2.0 parts by weight of Olivem 2020 (emulsifier), and 2.0 parts by weight of hexanediol were put into 72.0 parts by weight of purified water and stirred for 10 minutes at 1,000 rpm to prepare an aqueous phase lysate. Then, to prepare an oily phase lysate, 6.0 parts by weight of caprylic/capric triglyceride (oil), 6.0 parts by weight of cetyl ethylhexanoate (oil), and 1.0 parts by weight of Olivem 1000 (emulsifier) were dissolved while warming to 80 °C, and the prepared aqueous phase lysate was put thereinto and stirred at 1,000 rpm to prepare an oily phase lysate. Subsequently, 0.03 parts by weight of an anti-aging peptide, 0.03 parts by weight of nicotinamide riboside, 3.0 parts by weight of butylene glycol, 0.5 parts by weight of soybean lecithin, 0.7 parts by weight of structure (each of Examples 1 to 7), and 0.04 parts by weight of adenosine were further put thereinto and stirred at 60 °C for 5 minutes at 2,000 rpm. Finally, 0.1 parts by weight of perfume, 1 part by weight of *Scutellaria baicalensis Georgi* extract, 0.5 parts by weight of *Portulaca oleracea* extract, and 0.1 parts by weight of Jeju cherry blossom extract were put thereinto, and stirred at 50 °C for 5 minutes at 3,000 rpm to prepare cosmetic compositions, respectively.

**[Comparative Preparative Example 1]**

A cosmetic composition was prepared by performing the same process as in Preparative Example 7, except that the fluorescent protein liposome of Comparative Example 5 was applied instead of the structure of Example 7.

**[Experimental Example 4]**

To confirm the degree of skin absorption (penetration) of the cosmetic compositions obtained in Preparative Example 7 and Comparative Preparative Example 1, the tissue section of the pig skin obtained through the process of applying each cosmetic composition to the pig skin (Franz Cell Membrane, FCM, APURES Co., Ltd.) was melted at room temperature, and then a mounting solution (National Diagnostics Corporation) was applied thereto and observed with a fluorescence microscope (Nikon Corporation, ECLIPSE TS2-FL), and the results are shown in FIG. 6. At this time, the tissue section was obtained by repeating the process of spreading 0.25 g of each cosmetic composition on pig skin (2×2 cm) four times at 10 minute intervals, standing it at room temperature for 24 hours, putting it into 2 ml of 3.7% formaldehyde (Sigma-Aldrich Co. Ltd.) for 20 minutes to fix it, washing it three times with 1X PBS, placing it in an OCT (optimal cutting temperature compound, Sakura Finetek Co., Ltd.), freezing it at -20 °C, and cutting it to a thickness of

15 μm with cryostat microtome (Leica Biosystems Co., Ltd.).

Referring to FIG. 6, it could be confirmed that Comparative Preparative Example 1 did not show a fluorescent protein in the transdermal/dermis of the skin, whereas Preparative Example 7 showed a fluorescent protein in the transdermal/dermis of the skin. This can be seen as a result of supporting that the cosmetic compositions according to the present invention are well absorbed (penetrated) by the skin.

**[Experimental Example 5]**

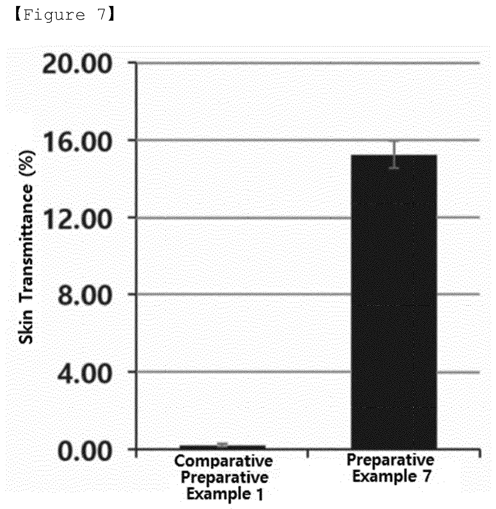

To confirm the degree of skin absorption (penetration) of the cosmetic compositions obtained in Preparative Example 7 and Comparative Preparative Example 1, 150 μl of the pig skin sample obtained through the process of applying each cosmetic composition to the pig skin (Franz Cell Membrane, FCM, APURES Co., Ltd.) was placed in a 96-well plate and the skin transmittance was analyzed with a fluorescence spectrophotometer (SpectraMAX M2, Molecular Devices, LLC), and the results are shown in FIG. 7. At this time, the pig skin sample was obtained by repeating the process of spreading 0.25 g of each cosmetic composition on pig skin (2×2 cm) four times at 10 minute intervals, and then repeating three times the process of soaking it in 1.5 ml of PBS, standing it at room temperature for 24 hours, and allowing the skin-absorbed (penetrated) fluorescent protein to be dissociated in PBS.

Referring to FIG. 7, it could be confirmed that the skin transmittance of Preparative Example 7 (15.25%) was much higher than that of Comparative Preparative Example 1 (0.21%). This can be seen as a result of supporting that the cosmetic composition according to the present invention is well absorbed (penetrated) by the skin.

**Claims**

1. A structure for accelerating transdermal absorption, comprising:

   an acicular body;
   a hydrophilic group binded to the acicular body; and
   a carrier binded to the hydrophilic group.

2. The structure for accelerating transdermal absorption according to claim 1, wherein the hydrophilic group is a hydroxyl group.

3. The structure for accelerating transdermal absorption according to claim 1, wherein the acicular body is a spicule extracted from porifera.

4. The structure for accelerating transdermal absorption according to claim 1, wherein the carrier is selected from the group consisting of a polymer hydrogel, a polymer micelle, a nanoemulsion, a liposome, an oleosome, a niosome, and an ethosome.

5. A method of manufacturing a structure for accelerating transdermal absorption, comprising the steps of:

   washing an acicular body;
   reacting the washed acicular body with a basic compound to bind a hydrophilic group to the acicular body; and
   mixing a carrier and the acicular body to which the hydrophilic group is binded, thereby binding the carrier to the hydrophilic group.

6. The method of manufacturing a structure for accelerating transdermal absorption according to claim 5, wherein the basic compound is one or more selected from the group consisting of sodium hydroxide, potassium hydroxide, and lithium hydroxide.

7. The method of manufacturing a structure for accelerating transdermal absorption according to claim 5, further comprising the step of reacting the washed acicular body with hydrogen peroxide solution to form pores in the acicular body.

8. The method of manufacturing a structure for accelerating transdermal absorption according to claim 7, wherein upon reaction of the washed acicular body and the hydrogen peroxide solution, ultrasonic waves having a frequency of 10 to 70 kHz and an output of 100 to 500 W are applied.

9. The method of manufacturing a structure for accelerating transdermal absorption according to claim 5, wherein upon mixing of the carrier and the acicular body to which the hydrophilic group is binded, a pressure of 50 to 300 MPa is applied for 1 to 10 minutes.

10. A cosmetic composition comprising:

the structure for accelerating transdermal absorption according to any one of claims 1 to 4; and
an active ingredient contained in the structure for accelerating transdermal absorption, wherein the active ingredient is one or more selected from the group consisting of nicotinamide adenine dinucleotide, nicotinamide mononucleotide, nicotinamide, nicotinamide riboside, retinol, retinyl acetate, retinyl palmitate, acetyl hexapeptide-3, pentapeptide-3, pentapeptide-18, palmitoyl tripeptide-1, palmitoyl pentapeptide-4, and hexapeptide-11.

【Figure 1】

【Figure 2】

Spicule Before Washing    Spicule After Washing

【Figure 3】

Example 2     Example 3     Example 4

Comparative
Example 1

【Figure 4】

Example 2     Example 3     Example 4

Example 5     Example 6     Example 7

【Figure 5】

Comparative Example 1 · Comparative Example 2 · Comparative Example 3 · Comparative Example 4

【Figure 6】

0 h     6 h     24 h

Comparative Preparative Example 1

0 h     6 h     24 h

Preparative Example 7

【Figure 7】

<table>
<tr><td colspan="2" align="center">**INTERNATIONAL SEARCH REPORT**</td><td>International application No.<br>**PCT/KR2020/004966**</td></tr>
</table>

**A.    CLASSIFICATION OF SUBJECT MATTER**

*A61K 8/02(2006.01)i, A61K 8/98(2006.01)i, A61K 8/14(2006.01)i, A61K 8/60(2006.01)i, A61K 8/67(2006.01)i, A61Q 19/00(2006.01)i*

According to International Patent Classification (IPC) or to both national classification and IPC

**B.    FIELDS SEARCHED**

Minimum documentation searched (classification system followed by classification symbols)

A61K 8/02; A61K 8/14; A61K 8/64; A61K 8/97; A61K 8/98; A61P 17/00; A61K 8/60; A61K 8/67; A61Q 19/00

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: spicule, hydrophilic, transdermal, cosmetic

**C.    DOCUMENTS CONSIDERED TO BE RELEVANT**

| Category* | Citation of document, with indication, where appropriate, of the relevant passages | Relevant to claim No. |
|---|---|---|
| X | KR 10-1862039 B1 (AMI COSMETIC CO., LTD.) 29 May 2018 | 1-6,9 |
| Y | See abstract; paragraphs [0014]-[0092], [0105] and [0106]; and claims 1-6 and 8. | 7,8,10 |
| Y | KR 10-1678621 B1 (KIM, Hyeong Kil) 22 November 2016<br>See paragraph [0028]; and claim 1. | 7,8,10 |
| A | KR 10-2018-0134468 A (IPIACOSMETIC.CO., LTD.) 19 December 2018<br>See entire document. | 1-10 |
| A | WO 2015-156455 A1 (PION-TECH CO., LTD et al.) 15 October 2015<br>See entire document. | 1-10 |
| PX | KR 10-2074214 B1 (HNB9 CO., LTD.) 06 February 2020<br>See claims 1-6 and 8-10. | 1-10 |
| | ※The above document is the published patent of a priority application of the present international application. | |

| ☐ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 21 JULY 2020 (21.07.2020) | **21 JULY 2020 (21.07.2020)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

| INTERNATIONAL SEARCH REPORT<br>Information on patent family members | | International application No.<br>**PCT/KR2020/004966** | |

| Patent document<br>cited in search report | Publication<br>date | Patent family<br>member | Publication<br>date |
| --- | --- | --- | --- |
| KR 10-1862039 B1 | 29/05/2018 | None | |
| KR 10-1678621 B1 | 22/11/2016 | KR 10-2016-0064410 A | 08/06/2016 |
| KR 10-2018-0134468 A | 19/12/2018 | None | |
| WO 2015-156455 A1 | 15/10/2015 | CN 106413723 A<br>KR 10-1760045 B1<br>KR 10-2015-0116620 A | 15/02/2017<br>20/07/2017<br>16/10/2015 |
| KR 10-2074214 B1 | 06/02/2020 | None | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20100104703
- KR 20110119062
- KR 20180134468